# EUROPEAN PATENT APPLICATION

(11) **EP 1 659 513 A2**
(43) Date of publication of application: **24.05.2006**
(21) Application number: 05257019.9
(22) Date of filing: 14.11.2005
(51) Int. Cl.: G06F 19/00

(54) **Method and apparatus for volume rendering display protocol**

(30) Priority: 23.11.2004 US 630435 P; 21.04.2005 US 111220
(71) Applicant: General Electronic Company, Schenectady, NY 12345 (US)
(72) Inventor: Kariathungal, Murali, Hollman Estates, Illinois 60194 (US); Yarger, Richard, Berkeley, Illinois 60163 (US); Driver, Tushad, Streamwood, Illinois 60107 (US); Jay, James, Lake Villa, Illinois 60046 (US)
(74) Representative: Pedder, James Cuthbert

(57) **Abstract**

A system and method to increase the efficiency and effectiveness of a radiologist is described. A display protocol for medical imaging equipment may allow a plurality of three-dimensional medical images to be concurrently displayed and navigated. The three-dimensional medical images may be linked so a user can manipulate the viewing angle and pointer in the linked images. The display protocol can be configured to display a three-dimensional image from a current exam and a three-dimensional comparison image. Moreover, a display protocol for medical imaging equipment may allow at least one three-dimensional medical image to be concurrently displayed with at least one two-dimensional medical image. The three-dimensional medical image may be linked with the two-dimensional medical image so a user can manipulate the viewing angle and pointer in the linked images. The display protocol can be configured to display a three-dimensional image with axial, sagital, coronal, or non-standard oblique views.

## Description

The present invention generally relates to a system and method for improved medical imaging. Particularly, the present invention relates to a more efficient system and method for configuring and interpreting medical images.

Medical diagnostic imaging systems encompass a variety of imaging modalities, such as x-ray systems, computerized tomography (CT) systems, ultrasound systems, electron beam tomography (EBT) systems, magnetic resonance (MR) systems, and the like. Medical diagnostic imaging systems generate images of an object, such as a patient, for example, through exposure to an energy source, such as x-rays passing through a patient, for example. The generated images may be used for many purposes. For instance, internal defects in an object may be detected. Additionally, changes in internal structure or alignment may be determined. Fluid flow within an object may also be represented. Furthermore, the image may show the presence or absence of objects in an object. The information gained from medical diagnostic imaging has applications in many fields, including medicine and manufacturing.

An example of a medical diagnostic imaging system is Picture Archival Communication Systems (PACS). PACS is a term for equipment and software that permits images, such as x-rays, ultrasound, CT, MRI, EBT, MR, or nuclear medicine for example, to be electronically acquired, stored and transmitted for viewing. Images from an exam may be viewed immediately or stored, or transmitted. The images may be viewed on diagnostic workstations by users, for example radiologists. In addition to viewing the images, the user may also view patient information associated with the image for example the name of the patient or the patient's sex.

The images acquired on a PACS system are generally two-dimensional datasets from imaging devices. A single exam may create thousands of two-dimensional images. Generally, a display protocol is used to control the organization and display of the two-dimensional images. For example, the display protocol may control the location and selection of the two-dimensional images displayed on the monitors when an exam is opened. The display protocol may also control whether the two-dimensional images are displayed in stack mode or displayed in a group. The display protocol may also control brightness, contrast, or whether any extra tools applied when the two-dimensional images are shown. Generally, the display protocol may control how a user views and interacts with the images.

As computer and medical imaging technology has become more sophisticated, the PACS diagnostic workstations have become capable of creating a reformatted three-dimensional model from the two-dimensional data sets. Currently, the three-dimensional models are of limited use to radiologists, at least partially because the display protocols for three-dimensional models are functionally limited and independent of each other. Thus, a need exists for a system and method that provides a more efficient and effective three-dimensional display protocol.

Moreover, current three-dimensional display protocols are independent of two-dimensional display protocols. Such independence creates confusion and difficulty for a user. A user wishing to view both three-dimensional and two-dimensional data of an exam may either have to go through considerable effort, or may not be able to do so at all. Thus, a need exists for a system and method that allows a user to view both two-dimensional data and three-dimensional data in an easier fashion.

Therefore, a need exists for a system and method that provides a display protocol for controlling the display and synchronization of three-dimensional data with other three-dimensional data and three-dimensional data with two-dimensional data. Such a system and method may allow a user to be more efficient and effective of diagnosis and treatment of medical conditions.

Certain embodiments of the present invention provide a system and method for displaying medical images. In an embodiment, the system for displaying medical images includes a computer unit for manipulating medical imaging data. The computer unit has a display protocol for configuring the medical imaging data for display as a plurality of volumetric medical images. The computer system for displaying medical images also includes at least one display unit for concurrently displaying the plurality of volumetric medical images to a user. In the computer system for displaying medial images the plurality of volumetric medical images may be linked. If the images are linked at least one of the volumetric medical images may be a driver image. The driver image may control the angle of view for other volumetric medical images. The driver image may also control the location of a pointer for other volumetric medical images. Moreover, at least one of the plurality of the volumetric medical images may be a current study. At least one of the plurality of volumetric medical images may also be a comparison study. The comparison study may be an archived study.

In an embodiment, a system for displaying medical images includes a computer unit for manipulating medical imaging data. The computer unit has a display protocol for configuring the medical imaging data for display as at least one volumetric medical image and at least one two-dimensional medical image. The system for displaying medical images also includes at least one display unit for concurrently displaying at least one volumetric medical image with at least one two-dimensional medical image to a user.

In the computer system for displaying medial images at least one volumetric medical image is linked to at least one two-dimensional medical image comprising the linked images. If the images are linked at least one of the medical images may be a driver image. The driver image may control the angle of view for other volumetric medical images. The driver image may also control the location of a pointer for other linked images. Moreover, at least one of the plurality of the volumetric medical images may be a current study. The two-dimensional medical images may comprise an axial image, a sagital image, a coronal image, or an oblique image.

In an embodiment, the method for displaying medical images includes configuring a display protocol. The display protocol including the ability to concurrently display a plurality of volumetric medical images and the ability to display at least one volumetric medical image concurrent with at least one two-dimensional medical image. The method for displaying medical images also includes selecting an exam or image for display, the selected exam or image being linked and displayed according to the display protocol. The method for displaying medical images also includes navigating the selected exam or image. The method of displaying medical images may further include at least one of said linked medical images is a driver image. The driver image may control the angle of view for other linked medical images. The driver image may also control the location of a pointer for other linked images.

In an embodiment, a computer - readable storage medium includes a set of instructions for a computer, the set of instructions for the computer including a configuration routine for configuring a display protocol, said display protocol including the ability to display a plurality of volumetric medical images concurrently and the ability to display at least one volumetric medical image concurrent with at least one two-dimensional medical image. The set of instructions also includes a selection routine for selecting an exam or image for display, said selected exam or image being linked and displayed according to said display protocol. The set of instructions also includes a navigation routine for navigating said selected exam or image.

The invention will now be described in greater detail, by way of example, with reference to the drawings, in which:-
Figure 1 illustrates a system using a display protocol comprising a plurality of volumetric images on separate display units in accordance with an embodiment of the present invention.
Figure 2 illustrates a system using a display protocol comprising a plurality of volumetric images on a single display unit in accordance with an embodiment of the present invention.
Figure 3 illustrates a system using a display protocol comprising at least one volumetric image and at least one two-dimensional image on separate display units in accordance with an embodiment of the present invention.
Figure 4 illustrates a system using a display protocol comprising at least one volumetric image and at least one two-dimensional image on a single display unit in accordance with an embodiment of the present invention.
Figure 5 illustrates a method in accordance with an embodiment of the present invention.
Figure 6 illustrates an example of a medical imaging system that may be used in accordance with an embodiment of the present invention.

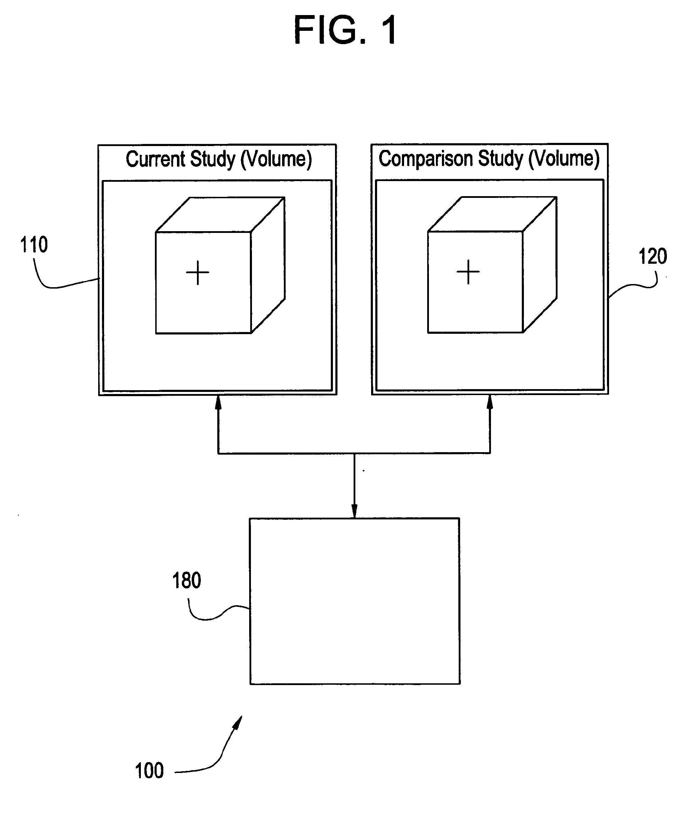

Figure 1 illustrates a system 100 for configuring and interpreting a three-dimensional volumetric display protocol in accordance with an embodiment of the present invention. The system 100 includes a display unit 110 and a display unit 120. In an embodiment, display unit 110 and display unit 120 may be two devices capable of electronic display, for example two computer monitors. In the embodiment shown in Figure 1, display unit 110 and display unit 120 may each display a single three-dimensional model, or volume. Although only two display units are shown, any number of display units may be used to display any number of volumes.

The system 100 also includes a computer unit 180. The computer unit 180 may be any equipment or software that permits electronic medical images, such as x-rays, ultrasound, CT, MRI, EBT, MR, or nuclear medicine for example, to be electronically acquired, stored, or transmitted for viewing and operation. The computer unit 180 may be connected to other devices as part of an electronic network. In an embodiment, the computer unit 180 may be, or may be part of, a picture archival communication system (PACS). In an embodiment, the system 100 is a PACS with display units 110 and 120 representing the display units of PACS. The computer unit 180 may represent other associated equipment. Alternatively, the computer unit 180 and display units 110 and 120 may be separate units. In such a case, the display unit 110 and a display unit 120 may be in electrical communication with the computer unit 180. The components of the system 100 may be single units, separate units, may be integrated in various forms, and may be implemented in hardware and/or in software.

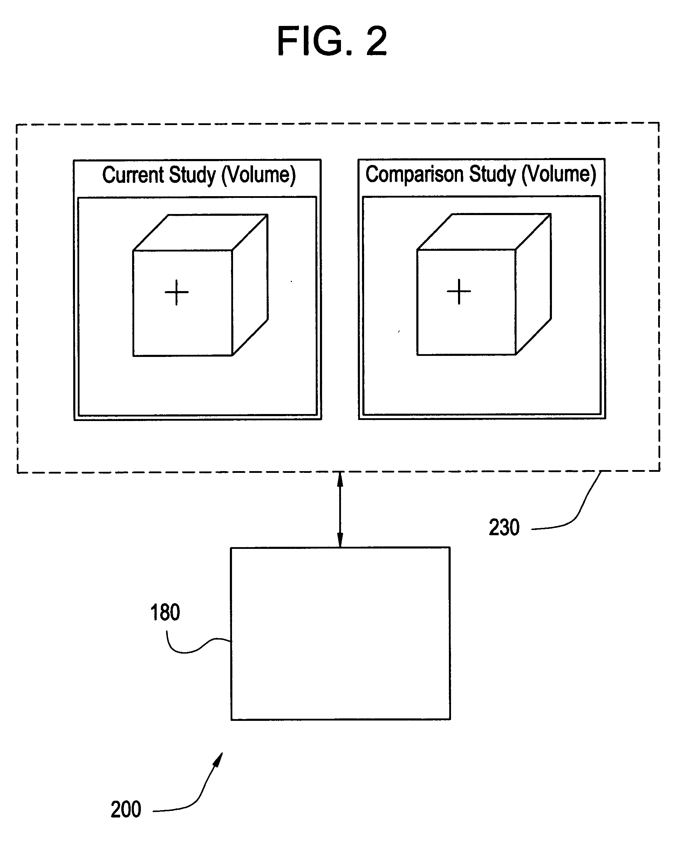

Similar to Figure 1, Figure 2 illustrates a system 200 for configuring and interpreting a volumetric display protocol in accordance with an embodiment of the present invention. The system 200 illustrates a single display unit 230. The display unit 230 may be capable of electronic display, for example a computer monitor. In the embodiment shown in Figure 2, display unit 230 displays two volumes, each volume located in a distinct portion of the display screen. Although only two volumes are shown, any number of volumes may be displayed on a single display unit. Also similar to Figure 1, The system 200 also includes computer unit 180. The computer unit 180 of the system 200 functions similarly to and has the same purpose as the computer unit 180 of the system 100.

Because system 100 and system 200 function similarly other than system 100 having a display unit for each volume and system 200 having a single display unit regardless of the number of volumes, the operational aspects of system 100 and 200 are described together. As mentioned above, the components of the systems 100 and 200 may be single units, separate units, may be integrated in various forms, and may be implemented in hardware and/or in software.

In an embodiment, a three-dimensional display protocol may allow a user to view multiple three-dimensional volumetric medical images concurrently and link the three-dimensional images for easier navigation and use. Allowing a user to view multiple three-dimensional images, or volume images, concurrently enables a user to easily compare the images. For example, a display protocol may instruct a volume image of a current study to be displayed on display unit 110 upon completion of an exam. The display unit 110 is labeled "Current Study (Volume)" as is one of the windows on display unit 230. A user may also use the display protocol to instruct a volume image of a comparison study to be displayed on display unit 120. The display unit 120 is labeled "Comparison Study (Volume)" as is one of the windows on display unit 230. In such a manner, the current study may be easily compared to the comparison study, providing a more efficient view for a user.

The volume image of a current study may be the image the user would like to examine. The volume image of a comparison study may be any image a user would like to compare to the current image. For example, the volume image of a current study may contain a set of organs from a current exam of patient A. A user may wish to view the same set of organs of patient A from a previous exam. A user may retrieve a comparison study from the computer unit 180, and display the comparison study on display unit 120 (or in window labeled "Comparison Study (Volume)" on display unit 230). In such an embodiment, the display unit 120 and corresponding window in display unit 230 is displaying an archived study. Additionally, the comparison study may be a volume from a different patient. The above are only examples, and any volumetric medical images may be compared.

Once the display protocol has displayed the desired images in the appropriate locations, the display protocol may link the volumetric medical images together. Generally, a volume may be manipulated to rotate the image, and thus rotate the angle of view for the user. In an embodiment of the invention, the display protocol links the volumetric medical images together so movement or rotation in one volume may drive similar movement or rotation in the other volumes. The image controlling the angle of view of the volumetric medical images is the driver image. Also, a cursor or pointer displayed on one volumetric medical image may point to a corresponding location on the other volumes. Similarly, the image controlling the location of the pointer on the volumetric medical images is the driver image.

In the embodiment of system 100 and 200, a user may select an image with a computer mouse, and use the computer mouse to rotate the image. The selected image may be the driver image. Rotation or movement in the driver image may drive rotation in the other images. For example, the user may select the current study on display unit 110 as the driver image. The user may then rotate or move the angle of view of the current study using the computer mouse. Because the display protocol has linked the comparison study on display unit 120, the comparison study may move or rotate to present an angle of view similar to the angle of view displayed for the current study. In such a manner, the user may view angle of view for both the current study and the comparison study. Moreover, a user may manipulate an on screen pointer to point to a specific location on the driver image. The display protocol may then manipulate the pointer on other images to point to a corresponding location. As a user moves the pointer in the driver image, the pointers in the other images correspondingly move to point to the same location. In such a manner, the user may be able to compare similar structures in multiple images more easily. The ability to link the volumetric medical images together increases the efficiency and accuracy of a user for diagnosis and treatment.

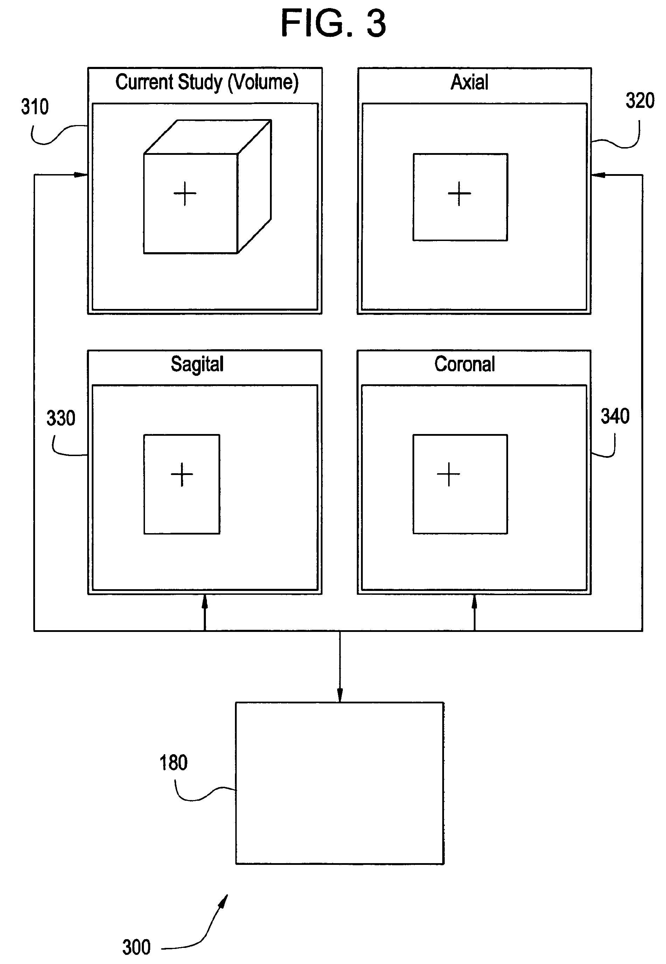

Figure 3 illustrates a system 300 for configuring and interpreting a display protocol that provides for display of volumetric and two-dimensional medical images in accordance with an embodiment of the present invention. The system 300 includes a display unit 310, a display unit 320, a display unit 330, and a display unit 340. In an embodiment, display unit 310, display unit 320, display unit 330, and display unit 340 may be separate devices capable of electronic display, for example computer monitors. In the embodiment shown in Figure 3, display unit 310, display unit 320, display unit 330, and display unit 340 each display a single image. Although four display units are shown, any number of display units may be used to display any number of images. The system 300 also includes computer unit 180. The computer unit 180 is similar to the computer unit 180 of systems 100 and 200 and has similar functions and operations.

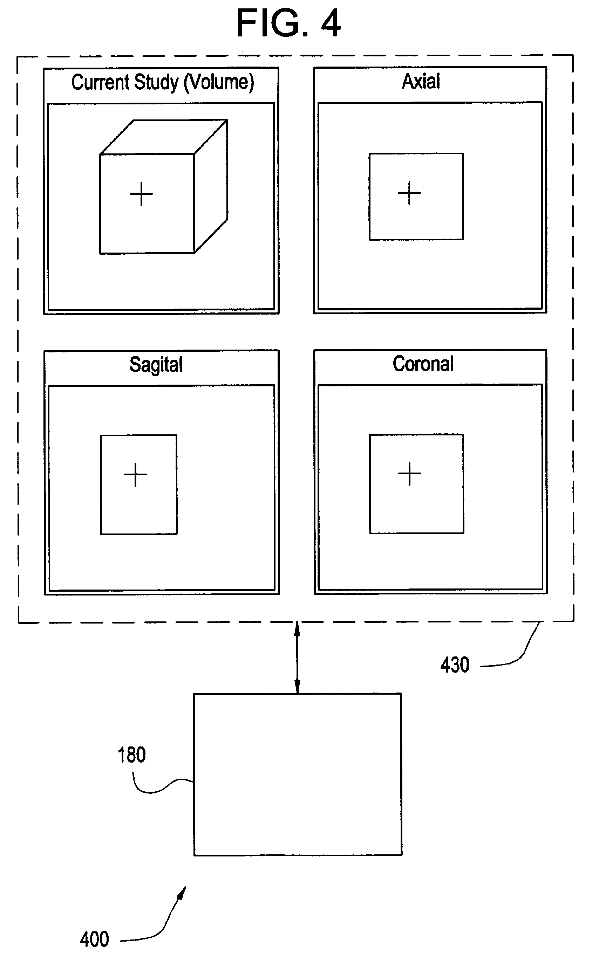

Similar to Figure 3, Figure 4 illustrates a system 400 for configuring and interpreting a display protocol that provides for display of volumetric and two-dimensional medical images in accordance with an embodiment of the present invention. The system 400 illustrates a single display unit 430. The display unit 430 may be capable of electronic display, for example a computer monitor. In the embodiment shown in Figure 4, display unit 430 displays four views. One view is a three-dimensional volumetric view, and the other three views are two-dimensional. Although only four views are shown, any number of views may be displayed on a single display unit. Also similar to Figure 3, the system 400 also includes computer unit 180. The computer unit 180 of the system 400 functions similarly to and has the same purpose as the computer unit 180 of the systems 100, 200, and 300.

Because system 300 and system 400 function similarly, other than system 300 having a display unit for each view and system 400 having a single display unit regardless of the number of views, the operational aspects of system 300 and 400 are described together. The components of the systems 300 and 400 may be single units, separate units, may be integrated in various forms, and may be implemented in hardware and/or in software.

In an embodiment, a display protocol may allow a user to view at least one three-dimensional volumetric medical image concurrently with at least one two-dimensional medical image and link the three-dimensional medical image to the two-dimensional image for easier navigation and use. Allowing a user to view a volume image concurrently with at least one two-dimensional image enables a user to easily compare the images. For example, a display protocol may instruct a volume image of a current study to be displayed on display unit 310, or in the case of the system 400 on the portion of the display 430 designated for display of the current study, upon completion of an exam. The display unit 310 and corresponding portion of display unit 430 is labeled "Current Study (Volume)." A user may also use the display protocol to display various two dimensional views upon completion of an exam. In the embodiments shown in Figures 3 and 4, the display protocol may display the axial, sagital, and coronal views of a current exam upon completion of the exam. In the embodiment of Figure 3, the axial, sagital, and coronal views may be displayed on display units 320, 330, and 340 respectively. In the embodiment of Figure 4, the axial, sagital, and coronal views may be displayed on dedicated portions of the screen of display unit 430. In such a manner, the current study may be easily compared to the comparison study, providing a more efficient tool to a user.

The volume image of the current study may be the three-dimensional image the user would like to examine. The axial, sagital, and coronal views may be the two-dimensional images corresponding the objects shown as part of the volume of the current study. Accordingly, a user may read a volume view of objects concurrently with various two-dimensional views of the same or similar objects.

For example, the volume image of a current study may contain a set of organs from a current exam of patient A. A user may wish to view the axial, sagital, and coronal views of the same set of organs of patient A from the current exam. The display protocol may display a volume view of the current study as well as the axial, sagital, or coronal views of the current study. Additionally, the invention is not limited to displays of current studies. A previous study may be displayed in the same manner. Additionally, the display units 310 - 340 or 430 may display a mixture of volume and axial, sagital, or coronal images from different patients.

Moreover, the display protocol may also display two-dimensional oblique views of objects along with other two-dimensional views and at least one volume view. For example, instead of displaying the axial, sagital, and coronal views along with the volume view in Figures 3 and 4, the display protocol may display the volume view with three oblique views. The display protocol may also display a mix of the axial, sagital, and coronal views along with the oblique views. For example, the volume view may be displayed with the axial, sagital, and one oblique view, or any combination thereof. The more display units available, as in the case of Figure 3, or display windows available, as in the case of Figure 4, the more combinations of views exist.

The above display protocols are only examples, and any volume images may be displayed along with any two-dimensional images.

Once the display protocol has displayed the desired images in the appropriate locations, the display protocol may link the images together. Generally, a volume may be manipulated to move or rotate the image, and thus the angle of view for the user. In an embodiment of the invention, the display protocol links the volume with the two-dimensional images so rotation or movement in a driver image may drive similar rotation or movement in the other images. Also, a cursor or pointer displayed on one image may point to a corresponding location on the other images. Similarly, the image controlling the location of the pointer on the images is the driver image.

In the embodiment of system 300 and 400, a user may select a driver image with a computer mouse, and use the computer mouse to move or rotate the image. Rotation or movement in one image may drive movement or rotation in the other images. For example, the user may select the volume displayed in display unit 310 as the driver image. The user may then move or rotate the angle of view of the volume in display unit 310 using the computer mouse. Because the display protocol has linked the other views displayed on display units 320, 330, and 340 respectively, the two-dimensional views may move or rotate to present an angle of view similar to the angle of view displayed for the volume. In such a manner, the user may view the volumetric view and the two-dimensional views from the same angle.

Moreover, a user may manipulate an on screen pointer to point to a specific location on the driver image. The display protocol may then manipulate the pointer on other images to point to a corresponding location. As a user moves the pointer in the driver image, the pointers in the other images correspondingly move to point to the same location. In such a manner, the user may be able to compare similar structures in multiple images more easily. The ability to link volumes with two-dimensional images increases the efficiency and accuracy of a user for diagnosis and treatment.

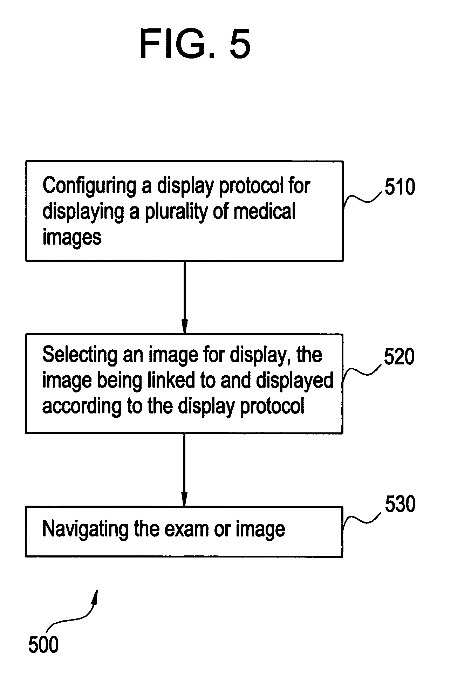

Figure 5 illustrates a method 500 in accordance with an embodiment of the present invention. At step 510, a user may customize the display protocol or use the factory default display protocol for displaying at least two volumetric medical images concurrently as well as displaying at least one volumetric medical image concurrent with at least one two-dimensional medical image. At step 520, a user may select an exam or image for display. The selected exam or image is linked and displayed according to the display protocol. At step 530, a user may navigate the selected exam or images.

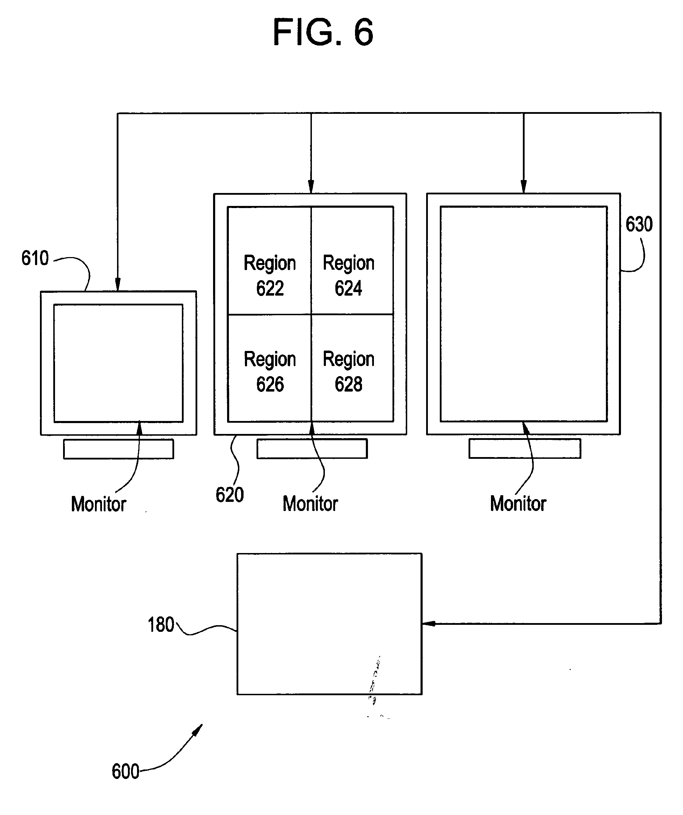

Figure 6 illustrates a system 600, which is example of a medical imaging system which may be used in accordance with an embodiment of the present invention. As an example, the system 600 shows three display units, unit 610, unit 620, and unit 630. The display units may be, for example, computer monitors. Units 610 and 630 include a single display region. Unit 620 includes four display regions, 622, 624, 626, and 628, respectively. Also included in the system 600 is the computer unit 180 which has the same functions as in systems 100-400 described above. Figure 6 is just an example, and a greater number or lesser number of displays may be used in accordance with the present invention. Moreover, any combination of images may be displayed in accordance with the present invention. The components of the system 600 may be single units, separate units, may be integrated in various forms, and may be implemented in hardware and/or in software.

In this example, display units 610 and 630 may correspond to display units 110 and 120 of system 100. Also, display unit 620 may correspond to display unit 430 in system 400. Accordingly, a user may chose a display protocol that displays a current study volume for display unit 610 and a comparison study volume for display unit 630, although any combination of volumetric medical images may be used. Moreover, a user may choose a display protocol that displays a volumetric medical image on region 622, an axial view of the medical image on region 624, a sagital view of the medical image on region 626, and a coronal view of the medical image on region 628. A user may also choose to display an oblique view in one of the regions, 622-628. Any combination of volumetric medical images and two-dimensional medical image may be used.

Continuing with the example in Figure 6, in any of the above display protocols, the images may be linked together. In the example shown in Figure 6, a user may alter the view, in an example, by clicking on the display unit or region and using a computer mouse or any other input device to rotate or change the view available to the user. For example, the user may click on the display unit 610, making the display unit 610 the driver image, moving or rotating some or all of the other views, for example. Any of the display units or regions may be the driver image. Also in the example of Figure 6, a cursor or pointer may appear in each image. As the images are linked, movement of the pointer in the driver image may move the pointer in the other images. The user may point to an object in one of the images, and the pointer may move to the object in the other images. Any of the display units or regions may be the driver image.

The system and method described above may be carried out as part of a computer - readable storage medium including a set of instructions for a computer. The set of instructions includes a configuration routine for configuring a display protocol. The display protocol includes the ability to display a plurality of volumetric medical images concurrently and the ability to display at least one volumetric medical image concurrent with at least one two-dimensional medical image. The set of instructions also includes a selection routine for selecting an exam or image for display. The selected exam or image being is linked and displayed according to the display protocol. The set of instructions also includes a navigation routine for navigating the selected exam or image.

## Claims

1. A system (100-400, 600) for displaying medical images, said system comprising:
a computer unit (180) for manipulating medical imaging data, said computer unit having computer software operating a display protocol for configuring said medical imaging data for display as a plurality of volumetric medical images, and;
at least one display unit (110, 120, 230, 310-340, 430, 610-630) for concurrently displaying said plurality of volumetric medical images to a user.

2. The system of claim 1, wherein the plurality of volumetric medical images are linked.

3. The system of claim 2, wherein at least one of said volumetric medical images is a driver image.

4. The system of claim 3, wherein said driver image controls the angle of view for other volumetric medical images.

5. The system of claim 3, wherein said driver image controls the location of a pointer for other volumetric medical images.

6. A system (100-400, 600) for displaying medical images, said system comprising:
a computer unit (180) for manipulating medical imaging data, said computer unit having computer software operating a display protocol for configuring said medical imaging data for display as at least one volumetric medical image and at least one two-dimensional medical image, and;
at least one display unit (110, 120, 230, 310-340, 430, 610-630) for concurrently displaying at least one volumetric medical image with at least one two-dimensional medical image to a user.

7. The system of claim 6, wherein at least one volumetric medical image is linked to at least one two-dimensional medical image comprising the linked images.

8. The system of claim 7, wherein at least one of said linked images is a driver image.

9. A method (500) for displaying medical images, said method comprising:
configuring (510) a display protocol, said display protocol including the ability to concurrently display a plurality of volumetric medical images and the ability to display at least one volumetric medical image concurrent with at least one two-dimensional medical image;
selecting (520) an exam or image for display, said selected exam or image being linked and displayed according to said display protocol, and;
navigating (530) said selected exam or image.

10. A computer - readable storage medium including a set of instructions for a computer, the set of instructions comprising:
a configuration routine for configuring a display protocol, said display protocol including the ability to concurrently display a plurality of volumetric medical images and the ability to display at least one volumetric medical image concurrent with at least one two-dimensional medical image;
a selection routine for selecting an exam or image for display, said selected exam or image being linked and displayed according to said display protocol, and;
a navigation routine for navigating said selected exam or image.
